# EUROPEAN PATENT SPECIFICATION

(11) **EP 4 015 044 B1**
(45) Date of publication and mention of the grant of the patent: **29.05.2024**
(21) Application number: 20858771.7
(22) Date of filing: 25.08.2020
(51) Int. Cl.: A61P 43/00, C07D 279/28, C07D 401/12, A61K 31/4439

(54) **METHOD FOR PREPARING ENANTIOMER OF SULFOXIDE COMPOUND, AND SYSTEM FOR PREPARING ENANTIOMER**
VERFAHREN ZUR HERSTELLUNG EINES ENANTIOMERS EINER SULFOXIDVERBINDUNG UND SYSTEM ZUR HERSTELLUNG EINES ENANTIOMERS
PROCÉDÉ DE PRÉPARATION D'UN ÉNANTIOMÈRE DE COMPOSÉ SULFOXYDE ET SYSTÈME DE PRÉPARATION D'UN ÉNANTIOMÈRE

(30) Priority: 29.08.2019 JP 2019156444
(43) Date of publication of application: 22.06.2022
(73) Proprietor: Tokyo University of Science Foundation, Tokyo 162-8601 (JP)
(72) Inventor: TAKAHASHI Hideyo, Tokyo 162-8601 (JP); MAKINO Kosho, Tokyo 162-8601 (JP); GU Kaya, Tokyo 162-8601 (JP); TANAKA Yuki, Tokyo 162-8601 (JP)
(74) Representative: Isarpatent
(86) International application number: PCT/JP2020/032053
(87) International publication number: WO 2021/039800

(56) References cited:
- JP-A- 2009 502 906
- JP-A- 2009 542 624
- CIRILLI ET AL: "High-performance liquid chromatography enantioseparation of proton pump inhibitors using the immobilized amylose-based Chiralpak IA chiral stationary phase in normal-phase, polar organic and reversed-phase conditions", JOURNAL OF CHROMATOGRAPHY A, ELSEVIER, AMSTERDAM, NL, vol. 1177, no. 1, 17 November 2007 (2007-11-17), pages 105-113, XP022392455, ISSN: 0021-9673, DOI: 10.1016/J.CHROMA.2007.11.027
- CHENNURU LAKSHMI NARAYANA ET AL: "Enantiomeric separation of proton pump inhibitors on new generation chiral columns using LC and supercritical fluid chromatography : Liquid Chromatography", JOURNAL OF SEPARATION SCIENCE, vol. 36, no. 18, 12 August 2013 (2013-08-12), pages 3004-3010, XP055960128, DE ISSN: 1615-9306, DOI: 10.1002/jssc.201300419 Retrieved from the Internet: URL:https://api.wiley.com/onlinelibrary/td m/v1/articles/10.1002%2Fjssc.201300419>
- GAITANI C M D ET AL: "Degradation and configurational changes of thioridazine 2-sulfoxide", JOURNAL OF PHARMACEUTICAL AND BIOMEDICAL ANALYSIS, ELSEVIER B.V, AMSTERDAM, NL, vol. 36, no. 3, 15 November 2004 (2004-11-15), pages 601-607, XP004620000, ISSN: 0731-7085, DOI: 10.1016/J.JPBA.2004.07.011
- ERLANDSSON P ET AL: "RESOLUTION OF THE ENANTIOMERS OF OMEPRAZOLE AND SOME OF ITS ANALOGUES BY LIQUID CHROMATOGRAPHY ON A TRISPHENYLCARBAMOYLCELLULOSE-BASED STATIONARY PHASE THE EFFECT OF THE ENANTIOMERS OF OMEPRAZOLE ON GASTRIC GLANDS", JOURNAL OF CHROMATOGRAPHY B, ELSEVIER SCIENCE PUBLISHERS, NL, vol. 532, 1 January 1990 (1990-01-01), pages 305-319, XP000828072, ISSN: 0378-4347, DOI: 10.1016/S0378-4347(00)83781-0
- "Method and Results", Annual Meeting of the Pharmaceutical Society of Japan. 27PA-am001S, page 20180000,
- MISLOW Kurt, Axelrod Michael, Rayner Dennis R.: "Light-Induced Pyramidal Inversion of Sulfoxides", Journal of the American Chemical Society, vol. 87, 1965, pages 4958-4959, XP055797594,
- Eap C. B., Souche A., Koeb L., Baumann P.: "Light-Induced Racemization Artifacts in the Analysis of the Diastereoisomeric Pairs of Thioridazine 5-Sulfoxide in the Plasma and Urine of Patients Treated with Thioridazine", THERAPEUTIC DRUG MONITORING, vol. 13, no. 4, 30 November 1990 (1990-11-30), pages 356-362, XP009534630, NEW YORK, NY, US ISSN: 0163-4356
- AURISICCHIO Claudia, Baciocchi Enrico, Gerini Maria Francesca, Lanzalunga Osvaldo: "Thermal and Photochemical Racemization of Chiral Aromatic Sulfoxides via the Intermediacy of Sulfoxide Radical Cations", Organic Letters, vol. 9, no. 10, February 2007 (2007-02), pages 1939-1942, XP055797599, DOI: 10.1021/ol070500y
- LEEK HANNA ET AL: "Preparative Scale Resolution of Enantiomers Enables Accelerated Drug Discovery and Development", MOLECULES, vol. 22, no. 1, 18 January 2017 (2017-01-18), page 158, XP093026775, DOI: 10.3390/molecules22010158
- CANNAZZA GIUSEPPE ET AL: "On-line racemization by high-performance liquid chromatography", JOURNAL OF CHROMATOGRAPHY A, vol. 1216, no. 30, 1 July 2009 (2009-07-01), pages 5655-5659, XP093026671, AMSTERDAM, NL ISSN: 0021-9673, DOI: 10.1016/j.chroma.2009.05.057

## Description

### TECHNICAL FIELD

The present invention relates to a method for preparing an enantiomer, in which an enantiomer mixture of a sulfoxide compound having the sulfur atom in a sulfoxide group as an asymmetric center is optically resolved to selectively prepare one enantiomer, and an enantiomer preparation system, which is used in the method for preparing an enantiomer.

### BACKGROUND ART

As a proton pump inhibitor, sulfoxide compounds such as omeprazole and lansoprazole have been known so far. These sulfoxide compounds have an asymmetric center at the sulfur atom in a sulfoxide group, and *S*- and *R*-enantiomers exist based on the configuration of this asymmetric center.

The enantiomers of the above-described sulfoxide compounds are known to have e.g. different pharmacokinetics. Esomeprazole, the *S*-form of omeprazole, for example, is known to have smaller changes in pharmacokinetics and pharmacological actions between individuals than omeprazole, a racemate. In addition, dexlansoprazole, the *R*-form of lansoprazole, is known to have better stability to drug-metabolizing enzymes and pharmacokinetics than lansoprazole, a racemate.

A variety of methods for efficiently obtaining a desired enantiomer from a sulfoxide compound, a racemate, have been proposed under such background (see e.g. Patent Documents 1 to 3) .

However, a sulfoxide compound, a racemate, is a mixture of equal amounts of *S-* and *R*-forms, and thus a desired enantiomer is obtained only in a yield of 50% at most by the methods described in Patent Documents 1 to 3, and the remaining enantiomer has been wasted.

Meanwhile, it has been reported that an optically active sulfoxide compound is racemized by light irradiation (photoracemization) (see e.g. Non-Patent Documents 1 and 2).
Patent Document 1: Japanese Unexamined Patent Application (Translation of PCT Application), Publication No. 2009-502906
Patent Document 2: Japanese Unexamined Patent Application (Translation of PCT Application), Publication No. 2009-542624
Patent Document 3: Japanese Unexamined Patent Application (Translation of PCT Application), Publication No. H07-509499

Non-Patent Document 1: K. Mislow et al., J. Am. Chem. Soc., 1965, 87(21), pp. 4958-4959
Non-Patent Document 2: Kimura Tsubasa et al., "Photoracemization of sulfoxide in drug molecules", Proceedings of the 138th Annual Meeting of the Pharmaceutical Society of Japan, March 2018, 27PA-am001S

Cirilli R. et al., Journal of Chromatography A, 1177(1), 2007, pp. 105-113 describes the result of an investigation of the chiral resolving ability of the amylose-based Chiralpak IA chiral stationary phase towards omeprazole and other proton pump inhibitors under reversed-phase conditions.

Chennuru Lakshmi Narayana et al., Journal of Separation Science, 36(18), 2013, pp. 3004-3010 discloses studies of the the enantioselectivity of proton pump inhibitors using new generation chiral packaging materials.

Gaitani C M D et al., Journal of Pharmaceutical and Biomedical Analysis, 36(3), 2004, pp. 601-607 discusses studies of the degradation and epimerization of THD 2-SO in human plasma, buffer and methanolic solutions.

Erlandsson P. et al., Journal of Chromatography B, 532, 1990, pp- 305-319 discloses separation of the enantiomers of omeprazole and some of its analogues on a chiral stationary phase comprising trisphenylcarbamoylcellulose coated on 3-aminopropyl silica.

In "Methods and Results-Photoracemization in Drug Molecule", Annual Meeting of the Pharmaceutical Society of Japan. 27PA-am001S, p. 20180000, methods and results of photoracemization in drug molecules are reported.

Eap C.B. et al., Therapeutic Drug Monitoring, 13(4), 1990, pp. 356-362 discusses light-induced racemization artifacts in the analysis of the diastereomeric pairs of thioridazine 5-sulfoxidein the plasma and urine of patients treated with thioridazine.

Aurisicchio C. et al., Organic Letters, 9(19), 2007, pp. 1939-1942 discusses thermal and photochemical racemization of chiral aromatic sulfoxides via the intermediacy of sulfoxide radical cations, wherein efficient racemization of enantiomerically pure methyl aryl sulfoxides was obtained by N-methylquinolinium tetrafluoborate (NMQ⁺) sensitized photolysis and by one-electron oxidation catalyzed by tris(2,2'-bipyridyl)ruthenium(III) hexafluorophosphate.

Leek Hanna et al., Molecules, 22(1), 2017, p. 158 presents a number of challenging case studies of up-to-kilogram separation of racemic or enriched isomer mixtures using preparative liquid chromatography and super critical fluid chromatography.

Channazta G. et al., Journal of Chromatography A, 1216(30), 2009, pp. 5655-5659 reports development of an on-column stopped-flow bidimensional recycling HPLC procedure to obtain an enantiomeric enrichment starting from a racemic mixture.

### DISCLOSURE OF THE INVENTION

### Problems to be Solved by the Invention

A subject of the present invention is to provide a method for preparing an enantiomer, in which a desired enantiomer can be obtained in a high yield from an enantiomer mixture of a sulfoxide compound, and an enantiomer preparation system, which is used in the method for preparing an enantiomer.

### Means for Solving the Problems

The following embodiments are included in a specific method to solve the above subject.
<1> A method for selectively preparing one of enantiomers of a sulfoxide compound having a sulfur atom in a sulfoxide group as an asymmetric center in an enantiomer preparation system including an optical resolution part to optically resolve an enantiomer mixture of the sulfoxide compound into one enantiomer and the other enantiomer, and a light irradiation part to irradiate the other enantiomer obtained in the optical resolution part with light to racemize the enantiomer, the method including:
   Step A for optically resolving an enantiomer mixture of the sulfoxide compound in the optical resolution part into one enantiomer and the other enantiomer, isolating the one enantiomer and sending the other enantiomer to the light irradiation part,
   Step B for irradiating the other enantiomer obtained in Step A or Step C in the light irradiation part with light to racemize the enantiomer and sending the enantiomer mixture of the sulfoxide compound by racemization to the optical resolution part again with a recirculating system, and
   Step C for optically resolving the enantiomer mixture of the sulfoxide compound obtained in Step B into the one enantiomer and the other enantiomer in the optical resolution part, wherein the enantiomer mixture of the sulfoxide compound is optically resolved by a chromatography method using a chiral column in both Step A and Step C, and wherein Step B and Step C are repeated, wherein in Step B the other enantiomer is irradiated with light in the presence of a photosensitizer to racemize the enantiomer, wherein the photosensitizer is a solid photosensitizer immobilized to a carrier.
<2> The method for preparing an enantiomer according to <1>, wherein the chromatography method using a chiral column is a recycle HPLC using a chiral column.
<3> The method for preparing an enantiomer according to <2>, wherein in step B the other enantiomer coming from a column outlet of the chiral column of the recycle HPLC in step A is irradiated with light to photoracemize the other enantiomer, and the resulting enantiomer mixture is returned to a column inlet of the chiral column of the recycle HPLC.
<4> The method for preparing an enantiomer according to any one of <1> to <3>, wherein the sulfoxide compound is a compound represented by formula (1) below: 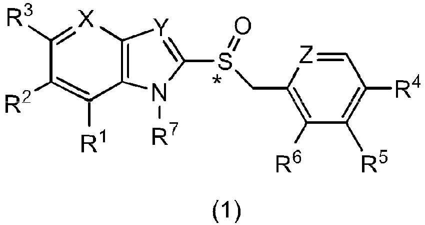
   wherein, R¹, R² and R³ are each independently a hydrogen atom, a halogen atom, a cyano group, a nitro group, a hydroxy group, a carboxy group, an alkyl group which may have a substituent, an alkoxy group which may have a substituent, an alkoxycarbonyl group, an aryl group, an aryloxy group, an acyl group, an acyloxy group or a heteroaryl group,
   R⁴, R⁵ and R⁶ are each independently a hydrogen atom, an alkyl group which may have a substituent, an alkoxy group which may have a substituent, or an amino group which may have a substituent,
   R⁷ is a hydrogen atom, a hydrocarbon group which may have a substituent, an acyl group or an acyloxy group,
   X, Y and Z are each independently a nitrogen atom or CH, and * represents an asymmetric center.
<5> An enantiomer preparation system for use in the method for preparing an enantiomer according to any one of <1> to <4>, the enantiomer preparation system including:
   an optical resolution part provided with a chiral column configured to optically resolve the enantiomer mixture of the sulfoxide compound into one enantiomer and the other enantiomer, and
   a light irradiation part configured to irradiate the other enantiomer obtained in the optical resolution part with light to racemize the enantiomer, wherein the optical resolution part optically resolves the enantiomer mixture of the sulfoxide compound obtained in the light irradiation part into the one enantiomer and the other enantiomer, further including a recirculating system which is configured to send an enantiomer mixture of the sulfoxide compound obtained in the light irradiation part to the optical resolution part again, wherein in the light irradiation part a solid photosensitizer immobilized to a carrier is present.
<6> The enantiomer preparation system according to <5>, wherein the optical resolution part provided with a chiral column is a recycle HPLC apparatus including a chiral column.

### Effects of the Invention

According to the present invention, it is possible to provide a method for preparing an enantiomer, in which a desired enantiomer can be obtained in a high yield from an enantiomer mixture of a sulfoxide compound, and an enantiomer preparation system, which is used in the method for preparing an enantiomer.

### BRIEF DESCRIPTION OF THE DRAWINGS

Fig. 1 is a drawing which shows an example of the schematic constitution of an enantiomer preparation system according to the present embodiment.
Fig. 2 is a graph which shows changes in enantiomeric excess with time when a photoracemization reaction of an optically active sulfoxide compound is allowed to proceed in the presence of a solid photosensitizer 2 in which TPT⁺, a photosensitizer, is covalently bound to a carrier.
Fig. 3 is a graph which shows changes in enantiomeric excess with time when a photoracemization reaction of an optically active sulfoxide compound is allowed to proceed in the presence of a solid photosensitizer 3 in which TPT⁺, a photosensitizer, is covalently bound to a carrier.

### PREFERRED MODE FOR CARRYING OUT THE INVENTION

### <Method for preparing enantiomer>

The method for selectively preparing an enantiomer according to the present embodiment is a method for preparing an enantiomer, in which one enantiomer of a sulfoxide compound having the sulfur atom in a sulfoxide group as an asymmetric center is selectively prepared in an enantiomer preparation system including an optical resolution part to optically resolve an enantiomer mixture of the sulfoxide compound into one enantiomer and the other enantiomer, and a light irradiation part to irradiate the other enantiomer obtained in the optical resolution part with light to racemize the enantiomer, the method including the following Step A to Step C:
Step A for optically resolving an enantiomer mixture of the sulfoxide compound in the optical resolution part into one enantiomer and the other enantiomer, isolating the one enantiomer and sending the other enantiomer to the light irradiation part;
Step B for irradiating the other enantiomer obtained in Step A or Step C in the light irradiation part with light to racemize the enantiomer and sending the enantiomer mixture of the sulfoxide compound by racemization to the optical resolution part again with a recirculating system; and
Step C for optically resolving the enantiomer mixture of the sulfoxide compound obtained in Step B into one enantiomer and the other enantiomer in the optical resolution part, wherein the enantiomer mixture of the sulfoxide compound is optically resolved by a chromatography method using a chiral column in both Step A and Step C, and wherein Step B and Step C are repeated, wherein in Step B the other enantiomer is irradiated with light in the presence of a photosensitizer to racemize the enantiomer, wherein the photosensitizer is a solid photosensitizer immobilized to a carrier.

### (Sulfoxide compound)

The sulfoxide compound is not particularly restricted as long as it is a compound which has an asymmetric center at the sulfur atom in a sulfoxide group and has *S-* and *R*-enantiomers based on the configuration of this asymmetric center. The sulfoxide compound is preferably a compound having only one asymmetric center from the viewpoint of efficiently preparing only a desired enantiomer.

A suitable sulfoxide compound is, for example, a compound represented by formula (1) below. The compound represented by formula (1) below has e.g. excellent gastric antisecretory, anti-ulcer, mucosal protective, anti-Helicobacter pylori actions, and is a compound useful as a proton pump inhibitor.

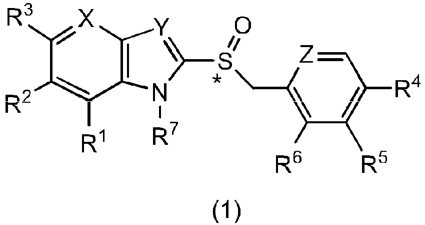

In the formula (1), R¹, R² and R³ are each independently a hydrogen atom, a halogen atom, a cyano group, a nitro group, a hydroxy group, a carboxy group, an alkyl group which may have a substituent, an alkoxy group which may have a substituent, an alkoxycarbonyl group, an aryl group, an aryloxy group, an acyl group, an acyloxy group or a heteroaryl group; R⁴, R⁵ and R⁶ are each independently a hydrogen atom, an alkyl group which may have a substituent, an alkoxy group which may have a substituent, or an amino group which may have a substituent; R⁷ is a hydrogen atom, a hydrocarbon group which may have a substituent, an acyl group or an acyloxy group; X, Y and Z are each independently a nitrogen atom or CH; and * represents an asymmetric center.

Examples of the halogen atom represented by R¹, R² and R³ include fluorine atom, chlorine atom, bromine atom, iodine atom and the like.

Examples of the "alkyl group" in the "alkyl group which may have a substituent" represented by R¹, R² and R³ include linear or branched C₁₋₇ alkyl groups such as a methyl group, an ethyl group, an n-propyl group, an isopropyl group, an n-butyl group, an isobutyl group, a sec-butyl group, a tert-butyl group, an n-pentyl group, an n-hexyl group and an n-heptyl group. Examples of the "substituent" in the "alkyl group which may have a substituent" include a halogen atom, a hydroxy group, C₁₋₆ alkoxy groups (e.g. a methoxy group, an ethoxy group, an n-propoxy group and an isopropoxy group), C₁₋₆ alkoxy-carbonyl groups (e.g. a methoxycarbonyl group, an ethoxycarbonyl group, an n-propoxycarbonyl group and an isopropoxycarbonyl group), a carbamoyl group and the like. The number of substituents is not particularly restricted, and may be, for example, one to three. When the number of substituents is two or more, the substituents may be the same or different.

Examples of the "alkoxy group" in the "alkoxy group which may have a substituent" represented by R¹, R² and R³ include linear or branched C₁₋₆ alkoxy groups such as a methoxy group, an ethoxy group, an n-propoxy group, an isopropoxy group, an n-butoxy group, an isobutoxy group, a sec-butoxy group, a tert-butoxy group, an n-pentoxy group and an n-hexyloxy group. Examples of the "substituent" in the "alkoxy group which may have a substituent" include the same as the above-described "substituent" in the "alkyl group which may have a substituent". The number of substituents is not particularly restricted and may be, for example, one to three. When the number of substituents is two or more, the substituents may be the same or different.

Examples of the "alkoxycarbonyl group" represented by R¹, R² and R³ include groups in which the alkoxy moiety is a linear or branched C₁₋₆ alkoxy group such as a methoxycarbonyl group, an ethoxycarbonyl group, an n-propoxycarbonyl group, an isopropoxycarbonyl group, an n-butoxycarbonyl group, an isobutoxycarbonyl group, a sec-butoxycarbonyl group, a tert-butoxycarbonyl group, an n-pentoxycarbonyl group and an n-hexyloxycarbonyl group.

Examples of the "aryl group" represented by R¹, R² and R³ include C₆₋₁₄ aryl groups such as a phenyl group, a 1-naphthyl group, a 2-naphthyl group, a biphenylyl group and a 2-anthryl group.

Examples of the "aryloxy group" represented by R¹, R² and R³ include C₆₋₁₄ aryloxy groups such as a phenyloxy group, a 1-naphthyloxy group, a 2-naphthyloxy group, a biphenylyloxy group and a 2-anthryloxy group.

Examples of the "acyl group" represented by R¹, R² and R³ include a formyl group; C₁₋₆ alkyl-carbonyl groups such as an acetyl group and a propionyl group; C₆₋₁₄ aryl-carbonyl groups such as a benzoyl group and a naphthalenecarbonyl group; and the like.

Examples of the "acyloxy group" represented by R¹, R² and R³ include C₁₋₆ alkyl-carbonyloxy groups such as an acetyloxy group and a propionyloxy group; C₆₋₁₄ aryl-carbonyloxy groups such as a benzoyloxy group and a naphthalenecarbonyloxy group; and the like.

Examples of the "heteroaryl group" represented by R¹, R² and R³ include 5-membered to 10-membered ring groups including one to three hetero atoms selected from nitrogen atom, sulfur atom and oxygen atom. Specific examples thereof include a 1-, 2- or 3-thienyl group, a 1-, 2-, 3- or 4-pyridyl group, a 2- or 3-furyl group, a 1-, 2- or 3-pyrrolyl group, a 2-, 3-, 4-, 5- or 8-quinolyl group, a 1-, 3-, 4- or 5-isoquinolyl group, a 1-, 2- or 3-indolyl group and the like.

Examples of the "alkyl group which may have a substituent" and "alkoxy group which may have a substituent" represented by R⁴, R⁵ and R⁶ include the same as the "alkyl group which may have a substituent" and "alkoxy group which may have a substituent" represented by R¹, R² and R³.

Examples of the "amino group which may have a substituent" represented by R⁴, R⁵ and R⁶ include an amino group; mono-C₁₋₆ alkylamino groups such as a methylamino group, an ethylamino group, an n-propylamino group and an isopropylamino group; mono-C₆₋₁₄ arylamino groups such as a phenylamino group, a 1-naphthylamino group and a 2-naphthylamino group; di-C₁₋₆ alkylamino groups such as a dimethylamino group, a diethylamino group, a methylethylamino group and a methylisobutylamino group; di-C₆₋₁₄ arylamino groups such as a diphenylamino group; and the like.

Examples of the "hydrocarbon group" in the "hydrocarbon group which may have a substituent" represented by R⁷ include C₁₋₁₉ hydrocarbon groups such as an alkyl group, an alkenyl group, an alkynyl group, an aryl group and an aralkyl group.

Examples of the alkyl group in R⁷ include linear or branched C₁₋₆ alkyl groups such as a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a neopentyl group, an n-hexyl group and an isohexyl group; cyclic C₃₋₁₄ alkyl groups such as a cyclopentyl group and a cyclohexyl group; and the like.

Examples of the alkenyl group in R⁷ include linear or branched C₂₋₆ alkenyl groups such as an allyl group, an isopropenyl group, an isobutenyl group, a 2-pentenyl group and a 2-hexenyl group; cyclic C₃₋₁₄ alkenyl groups such as a 2-cyclohexenyl group; and the like.

Examples of the alkynyl group in R⁷ include linear or branched C₂₋₆ alkynyl groups such as a propargyl group, a 2-butynyl group, a 3-butynyl group, a 3-pentynyl group and a 3-hexynyl group.

Examples of the aryl group in R⁷ include C₆₋₁₄ aryl groups such as a phenyl group, a 1-naphthyl group, a 2-naphthyl group, a biphenylyl group and a 2-anthryl group.

Examples of the aralkyl group in R⁷ include C₇₋₁₉ aralkyl groups such as phenyl-C₁₋₄ alkyl groups, e.g. a benzyl group, a phenethyl group and a phenylpropyl group; a benzhydryl group; and a trityl group.

When the hydrocarbon group represented by R⁷ is an alkyl group, an alkenyl group or an alkynyl group, the hydrocarbon group may be substituted by an alkylthio group (e.g. a C₁₋₄ alkylthio group such as a methylthio group, an ethylthio group, an n-propylthio group or an isopropylthio group), a halogen atom (e.g. fluorine atom, chlorine atom, bromine atom or iodine atom), an alkoxy group (e.g. a C₁₋₆ alkoxy group such as a methoxy group, an ethoxy group, an n-propoxy group or an isopropoxy group), an acyloxy group (e.g. a C₁₋₆ alkyl-carbonyloxy group such as an acetyloxy group or an n-propionyloxy group; a C₆₋₁₄ aryl-carbonyloxy group such as a benzoyloxy group or a naphthalenecarbonyloxy group), a nitro group, an alkoxycarbonyl group (e.g. a C₁₋₆ alkoxy-carbonyl group such as a methoxycarbonyl group, an ethoxycarbonyl group, an n-propoxycarbonyl group or an isopropoxycarbonyl group), an alkylamino group (e.g. a mono- or di-C₁₋₆ alkylamino group such as a methylamino group, an ethylamino group, an n-propylamino group, an isopropylamino group, a dimethylamino group, a diethylamino group, a methylethylamino group or a methylisobutylamino group), an alkoxyimino group (e.g. a C₁₋₆ alkoxy-imino group such as a methoxyimino group, an ethoxyimino group, an n-propoxyimino group or an isopropoxyimino group), a hydroxyimino group or the like. The number of substituents is not particularly restricted and may be, for example, one to three. When the number of substituents is two or more, the substituents may be the same or different.

In addition, when the above hydrocarbon group is an aryl group or an aralkyl group, the hydrocarbon group may be substituted by an alkyl group (e.g. a linear or branched C₁₋₆ alkyl group such as a methyl group, an ethyl group, an n-propyl group, an isopropyl group, an n-butyl group, an isobutyl group, a sec-butyl group, a tert-butyl group, an n-pentyl group or an n-hexyl group; a cyclic C₃₋₆ alkyl group such as a cyclohexyl group), an alkenyl group (e.g. a C₂₋₆ alkenyl group such as an allyl group, an isopropenyl group, an isobutenyl group, a 1-methylallyl group, a 2-pentenyl group or a 2-hexenyl group), an alkynyl group (e.g. a C₂₋₆ alkynyl group such as a propargyl group, a 2-butynyl group, a 3-butynyl group, a 3-pentynyl group or a 3-hexynyl group), an alkoxy group (e.g. a C₁₋₆ alkoxy group such as a methoxy group, an ethoxy group, an n-propoxy group or an isopropoxy group), an acyl group (e.g. a formyl group; a C₁₋₆ alkyl-carbonyl group such as an acetyl group or a propionyl group; a C₆₋₁₄ aryl-carbonyl group such as a benzoyl group or a naphthalenecarbonyl group), a nitro group, an amino group, a hydroxy group, a cyano group, a sulfamoyl group, a mercapto group, a halogen atom (e.g. fluorine atom, chlorine atom, bromine atom or iodine atom), an alkylthio group (e.g. a C₁₋₄ alkylthio group such as a methylthio group, an ethylthio group, an n-propylthio group or an isopropylthio group) or the like. The number of substituents is not particularly restricted and may be, for example, one to five. When the number of substituents is two or more, the substituents may be the same or different.

Examples of the "acyl group" and "acyloxy group" represented by R⁷ include the same as the "acyl group" and "acyloxy group" represented by R¹, R² and R³.

Among compounds represented by the above formula (1), preferred are compounds wherein R¹ is a hydrogen atom, R² is a hydrogen atom, a C₁₋₄ alkyl group which may be substituted by a halogen atom, a C₁₋₄ alkoxy group which may be substituted by a halogen atom, or a heteroaryl group (preferably a 1-, 2- or 3-pyrrolyl group), R³ is a hydrogen atom or a C₁₋₄ alkoxy-carbonyl group, R⁴ is a hydrogen atom, a C₁₋₄ alkyl group which may be substituted by a halogen atom, or a C₁₋₄ alkoxy group which may be substituted by a halogen atom or a C₁₋₄ alkoxy group, R⁵ is a hydrogen atom, or a C₁₋₄ alkoxy group which may be substituted by a halogen atom or a C₁₋₄ alkoxy group, R⁶ is a hydrogen atom, a C₁₋₄ alkyl group which may be substituted by a halogen atom, a C₁₋₄ alkoxy group which may be substituted by a halogen atom or a C₁₋₄ alkoxy group, or a di-C₁₋₄ alkylamino group, and R⁷ is a hydrogen atom.

Specific examples of the compound represented by the above formula (1) are shown below. However, the sulfonyl compound in the present embodiment is not limited to these examples.

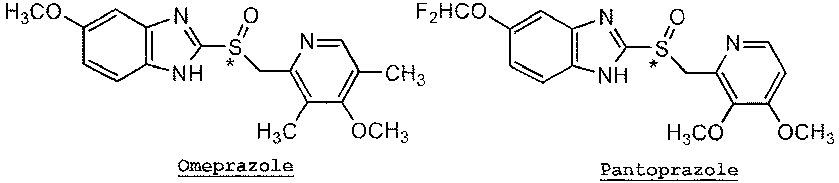

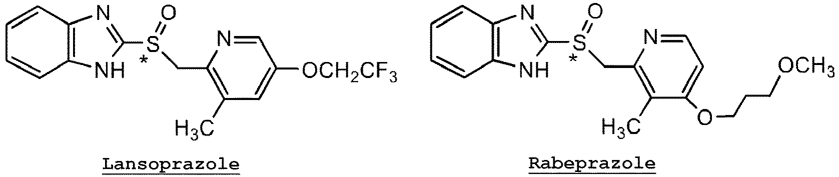

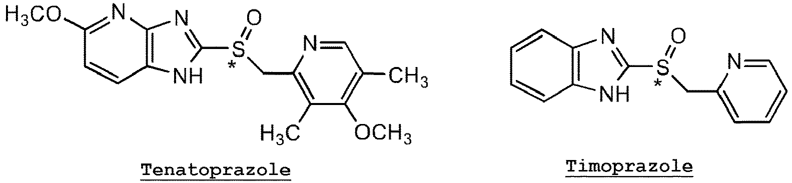

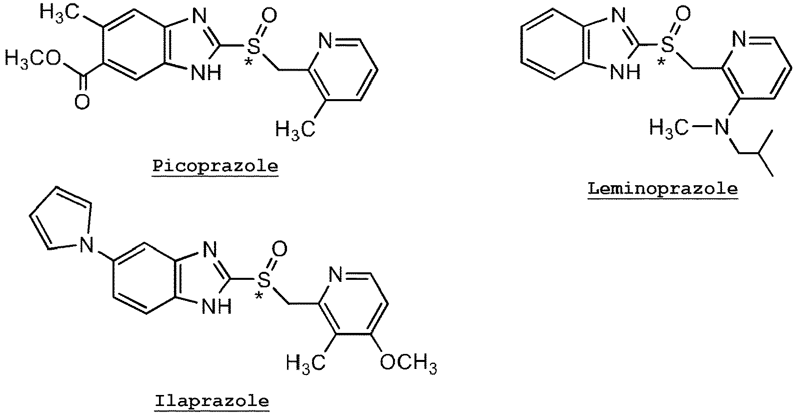

The compound represented by the above formula (1) may be in salt and hydrate forms. The salt is preferably a pharmaceutically acceptable salt, and examples thereof include a salt with an inorganic base, a salt with an organic base, a salt with a basic amino acid, and the like. Examples of the salt with an inorganic base include alkali metal salts such as sodium salt and potassium salt; alkali earth metal salts such as calcium salt and magnesium salt; ammonium salt; and the like. Examples of the salt with an organic base include salts with trimethylamine, triethylamine, pyridine, picoline, ethanolamine, diethanolamine, triethanolamine, dicyclohexylamine, N,N'-dibenzylethylenediamine and the like. Examples of the salt with a basic amino acid include salts with arginine, lysine, ornithine and the like.

### (Step A)

In Step A, an enantiomer mixture of a sulfoxide compound is optically resolved into one enantiomer and the other enantiomer.

The enantiomer mixture is not particularly restricted as long as it includes R- and S-forms, and is not required to be a racemate. The enantiomeric excess of the enantiomer mixture may be 0% ee to 25% ee, may be 0% ee to 10% ee, or may be 0% ee to 5% ee.

The optical resolution method is a chromatography method using a chiral column. The chromatography method using a chiral column is used in both Step A and Step C.

### (Step B)

In Step B, the other enantiomer obtained in Step A or Step C described below is irradiated with light to racemize (photoracemize) the enantiomer.

It has been reported that an optically active sulfoxide compound is racemized by light irradiation (see e.g. Non-Patent Document 1). Because of this, by irradiating the other enantiomer obtained in Step A or Step C with light, the enantiomer can be racemized.

The other enantiomer to be irradiated with light may be in a solid state or a state of being dissolved in a solvent, and the latter is preferred in terms of racemization efficiency. Using a chromatography method in Step A, the eluate including the other enantiomer can be irradiated with light.

It should be noted that a photosensitizer is used in Step B as described below, wherein the photosensitizer is a solid photosensitizer immobilized to a carrier.

It is preferred that the wavelength of irradiation light be properly adjusted depending on the type of the sulfoxide compound and the presence or absence of a sensitizer, and, for example, the wavelength may be 200 nm to 450 nm, may be 200 nm to 400 nm, or may be 254 nm to 365 nm. The time for light irradiation varies depending on e.g. the type of the sulfoxide compound, the wavelength of irradiation light, and the presence or absence of a sensitizer; however, in general, racemization can be sufficiently achieved by light irradiation for 5 minutes to 2 hours.

### (Step C)

In Step C, an enantiomer mixture of the sulfoxide compound obtained in Step B is optically resolved into one enantiomer and the other enantiomer. The enantiomeric excess of the enantiomer mixture obtained in Step B may be 0% ee to 25% ee, may be 0% ee to 10% ee, or may be 0% ee to 5% ee.

The optical resolution method in Step C is the same as the method in Step A. The chromatography method using a chiral column is used in both Step A and Step C.

By the above Step B and Step C, the yield of a desired enantiomer can be largely improved compared to a case where only Step A is carried out. The yield of a desired enantiomer is further improved by repeating the above Step B and Step C.

### (Photosensitizer)

In the above Step B, an optically active sulfoxide compound is irradiated with light in the presence of a photosensitizer. The efficiency of racemization is further increased by light irradiation in the presence of a photosensitizer.

The photosensitizer is not particularly restricted, and conventionally known photosensitizers can be used. Among photosensitizers, those with the maximum absorption wavelength in a wavelength range of 365 nm to 450 nm are preferred. Specific examples of the photosensitizer include cyanoarene compounds such as 1,2,4,5-tetracyanobenzene, 6,6'-dicyano-2,2'-bipyridyl, 1,2-dicyanonaphthalene and 9,10-dicyanoanthracene; pyrylium compounds such as 2,4,6-triphenylpyrylium salts and 2,4,6-triphenylthiopyrylium salts; acridinium compounds such as 10-methyl-9-(2,4,6-trimethylphenyl)acridinium salts and 3,6-diamino-10-methylacridinium salts; quinone compounds such as 2,3-dichloro-5,6-dicyano-p-benzoquinone and anthraquinone; quinolinium compounds such as 1-methylquinolinium salts and 1-methyl-6-methoxyquinolinium salts; xanthene compounds such as fluorescein and rhodamine B; thiazine compounds such as 10-phenylphenothiazine and methylthioninium salts; benzophenone compounds such as benzophenone and thioxanthone; and the like. Among these, cyanoarene compounds, pyrylium compounds, acridinium compounds, quinone compounds and quinolinium compounds are preferred.

The photosensitizer is a solid photosensitizer which is immobilized to a carrier such as silica gel. By using the solid photosensitizer, an enantiomer mixture of the sulfoxide compound obtained in Step B and the photosensitizer can be easily separated. The method for immobilizing a photosensitizer to a carrier is not particularly restricted, and may be a covalent bond or ion bond.

### <Enantiomer preparation system>

The enantiomer preparation system according to the present embodiment is an enantiomer preparation system which is used in the above-described method for preparing an enantiomer, and includes an optical resolution part provided with a chiral column configured to optically resolve an enantiomer mixture of the sulfoxide compound into one enantiomer and the other enantiomer, and a light irradiation part configured to irradiate the other enantiomer obtained in the optical resolution part with light to racemize the enantiomer, wherein the optical resolution part optically resolves the enantiomer mixture of the sulfoxide compound obtained in the light irradiation part into the one enantiomer and the other enantiomer, further including a recirculating system which is configured to send an enantiomer mixture of the sulfoxide compound obtained in the light irradiation part to the optical resolution part again, wherein in the light irradiation part a solid photosensitizer immobilized to a carrier is present.

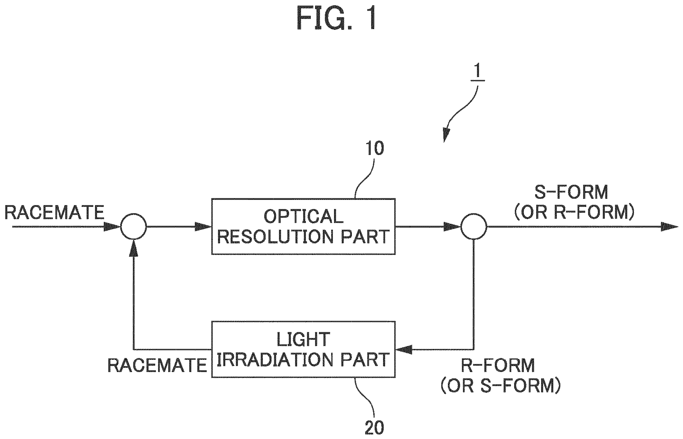

One example of the schematic constitution of the enantiomer preparation system according to the present embodiment is shown in Fig. 1. As shown in Fig. 1, the enantiomer preparation system 1 includes an optical resolution part 10 and a light irradiation part 20.

The optical resolution part 10 optically resolves, for example, an enantiomer mixture of a sulfoxide compound into one enantiomer (e.g. S-form) and the other enantiomer (e.g. R-form), and isolates one enantiomer and also sends the other enantiomer to the light irradiation part 20. The optical resolution part 10 is provided with a chiral column.

The light irradiation part 20 irradiates the other enantiomer obtained in the optical resolution part 10 (e.g. R-form) with light to racemize (photoracemize) the enantiomer. The light source of the light irradiation part 20 is not particularly restricted as long as the other enantiomer is irradiated with light at a wavelength required for racemization (e.g. light at a wavelength of 200 nm to 450 nm, preferably light at a wavelength of 254 nm to 365 nm). Specific examples of the light source include a high-pressure mercury lamp, a low-pressure mercury lamp, a xenon lamp, a LED lamp and the like. It should be noted that when a material which absorbs light from the light source or a material which changes the wavelength of light from the light source exists between the light source and the other enantiomer, it is preferred that the other enantiomer be irradiated with light in view of the absorption of light or changes in the wavelength of light. The light irradiation part 20 sends an enantiomer mixture of the sulfoxide compound obtained by racemization to the optical resolution part 10 again.

As described above, according to the enantiomer preparation system 1, the yield of a desired enantiomer can be largely improved by repeating optical resolution and photoracemization in the optical resolution part 10 and the light irradiation part 20.

It should be noted that a recirculating system, in which an enantiomer mixture of the sulfoxide compound obtained in the light irradiation part 20 is sent to the optical resolution part 10 again, is included in the above-described embodiment. The recycling system is preferred because a solvent can be reused and optical resolution and photoracemization are easily repeated.

### EXAMPLES

The present invention will now be described in more detail by way of examples thereof. It should be noted, however, that the present invention is not restricted to these examples.

### <Example 1: optical resolution of sulfoxide compound, racemate, comparative>

In Example 1, a compound, a racemate, represented by formula below was optically resolved using an enantiomer preparation system with the constitution shown in Fig. 1 to selectively prepare an S-compound.

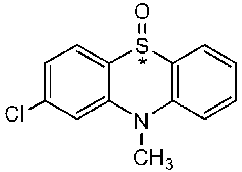

Specifically, the compound, a racemate, was optically resolved to isolate the S-compound by recycle HPLC using a chiral column, and the R-compound was returned from the column outlet to the column inlet. During the process of returning the R-compound to the column inlet, a region with a length of 200 cm in a channel with an internal diameter of 0.5 mm was irradiated with light to photoracemize the R-compound. The recycle HPLC conditions are as described below.

### -Recycle HPLC conditions-

Column: CHIRALPAK IC (10 mm x 250 mm, particle diameter 5 mm) + CHIRALPAK IH (10 mm x 250 mm, particle diameter 5 mm) (manufactured by Daicel Corporation),
Mobile phase: hexane/ethanol = 1/1,
Flow rate: 2.0 mL/min, and
Light irradiation part: LED lamp (wavelength 365 nm).

When the S-compound was isolated four times by repeating optical resolution and photoracemization, 4.3 mg (0.01634 mmol) of the S-compound was obtained from 5.0 mg of the compound, a racemate, (0.01900 mmol) (yield: 86%). The enantiomeric excess of the obtained S-compound was 96.4% ee.

### <Example 2: optical resolution of sulfoxide compound, racemate, comparative>

In Example 2, (±)-omeprazole, a racemate, was optically resolved to selectively prepare (S)-omeprazole.

Specifically, (S)-omeprazole and (R)-omeprazole were each isolated from 1.0 mL of a solution of (±)-omeprazole in acetonitrile (3.5 mg, 0.01 mmol, 0.01 M) by chiral HPLC. The chiral HPLC conditions are as described below.

### -Chiral HPLC conditions-

Column: CHIRALPAK IA (10 mm x 250 mm, particle diameter 5 mm) (manufactured by Daicel Corporation),
Mobile phase: hexane/ethanol = 1/1, and
Flow rate: 2.4 mL/min.

The solvent of (R)-omeprazole was distilled off, and acetonitrile was added thereto to prepare a 0.01 M acetonitrile solution. This acetonitrile solution was put in a quartz cell, and irradiated with light at a wavelength of 254 nm using a UV lamp for 10 minutes to photoracemize the (*R*)-omeprazole. After light irradiation, the solvent was distilled off, and (*S*)-omeprazole and (R)-omeprazole were each isolated again by chiral HPLC. Photoracemization and optical resolution are repeatedly carried out on (*R*)-omeprazole to isolate (S)-omeprazole and (*R*)-omeprazole. The obtained (*S*)-omeprazole were all combined and the solvent was distilled off to obtain 2.0 mg of (*S*)-omeprazole (0.00580 mmol) (yield 58%). The enantiomeric excess of the obtained (*S*)-omeprazole was 97.1% ee.

### <Reference Example 1: consideration of photosensitizer>

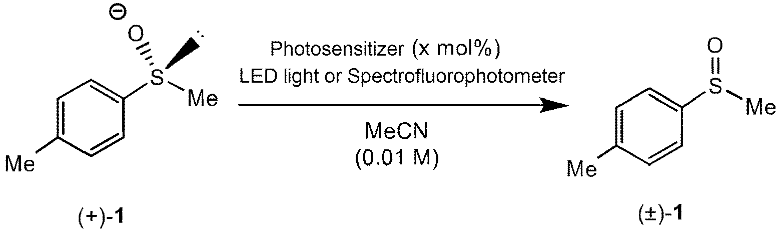

As shown in the above scheme, an optically active sulfoxide compound was irradiated with light in the presence of a variety of photosensitizers, and influences of photosensitizers on the photoracemization reaction were considered. The following 8 types of photosensitizers are used.

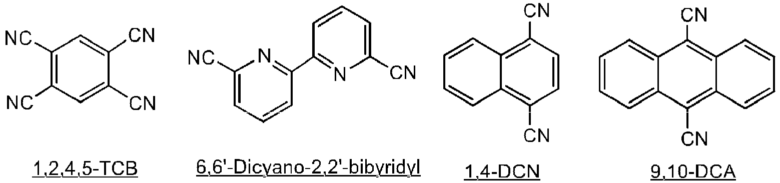

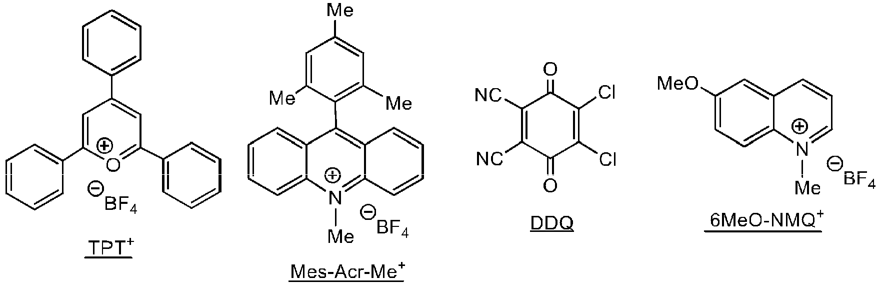

To an acetonitrile solution (2.0 mL, 0.01 M) containing an optically active sulfoxide compound ((+)-1) (3.08 mg, 0.02 mmol), a photosensitizer (0.02 mmol to 2 mmol, 0.1 mol% to 10 mol%) was added, and the obtained mixture was irradiated with light using an 18 W LED light (wavelength 365 nm to 425 nm) or a spectrofluorophotometer (wavelength 290 nm to 330 nm, RF-5300 PC manufactured by SHIMADZU CORPORATION). The same experiment was carried out without adding a photosensitizer for comparison. During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

**[Table 1]**

| Entry | Photosensitizer | Amount of added photosensitizer (mol%) | Light wavelength | Time required for racemization |
|---|---|---|---|---|
| 1 | 1,2,4,5-TCB | 10 | 330 nm | 15 min |
| 2 | 6,6'-Dicyano-2,2'-bipyridyl | 10 | 290 nm | 60 min |
| 3 | 1,4-DCN | 10 | 330 nm | 15 min |
| 4 | 9,10-DCA | 0.1 | 425 nm | 1 min |
| 5 | TPT⁺ | 1 | 425 nm | 0.5 min |
| 6 | Mes-Acr-Me⁺ | 1 | 425 nm | 6 min |
| 7 | DDQ | 10 | 380 nm | 2 min |
| 8 | 6MeO-NMQ⁺ | 1 | 365 nm | 2 min |
| 9 | - | - | 425 nm | - |

As shown in Table 1, in Entries 1 to 8 which was irradiated with light in the presence of a photosensitizer, the photoracemization reaction could be allowed to efficiently proceed. Contrarily, in Entry 9 in which a photosensitizer was not used, the photoracemization reaction did not proceed very well even by irradiation with light at a wavelength of 425 nm for 60 minutes.

### <Reference Example 2: consideration of solvent in photoracemization reaction using 9,10-DCA as photosensitizer>

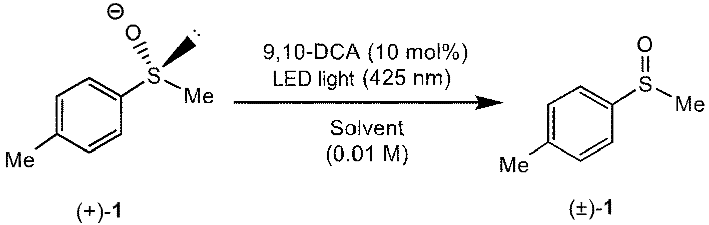

An optically active sulfoxide compound ((+)-1) (3.08 mg, 0.02 mmol) was dissolved in a variety of solvents (2.0 mL). To the obtained solution, 9,10-dicyanoanthracene (9,10-DCA) (0.45 mg, 2 mmol, 10 mol%) was added, and the obtained mixture was irradiated with light using an 18 W LED light (wavelength 425 nm). During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

**[Table 2]**

| Entry | Solvent | Time required for racemization |
|---|---|---|
| 1 | Acetonitrile | 0.5 min |
| 2 | Acetone | < 15 min |

As shown in Table 2, photoracemization could proceed even when any solvent was used, and the photoracemization reaction was fast particularly when acetonitrile was used.

### <Reference Example 3: consideration of solvent in photoracemization reaction using TPT⁺ as photosensitizer>

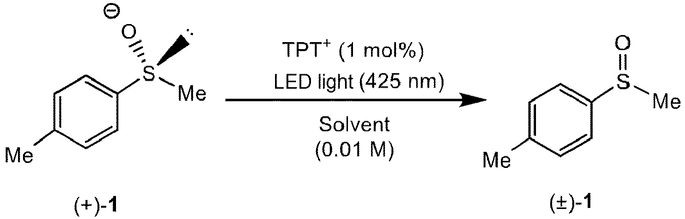

An optically active sulfoxide compound ((+)-1) (3.08 mg, 0.02 mmol) was dissolved in a variety of solvents (2.0 mL). To the obtained solution, 2,4,6-triphenylpyrylium tetrafluoroborate (TPT⁺) (80 mg, 0.2 mmol, 1 mol%) was added, and the obtained mixture was irradiated with light using an 18 W LED light (wavelength 425 nm). During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

**[Table 3]**

| Entry | Solvent | Time required for racemization | Enantiomer ratio after 10 min |
|---|---|---|---|
| 1 | Acetonitrile | 0.5 min | 50:50 |
| 2 | Toluene | > 10 min | 61:39 |
| 3 | Dichloromethane | < 10 min | 50:50 |
| 4 | Acetone | > 10 min | 56:44 |

As shown in Table 3, photoracemization could proceed even when any solvent was used, and the photoracemization reaction was fast particularly when acetonitrile was used.

### <Reference Example 4: consideration of solvent in photoracemization reaction using Mes-Acr-Me⁺ as photosensitizer>

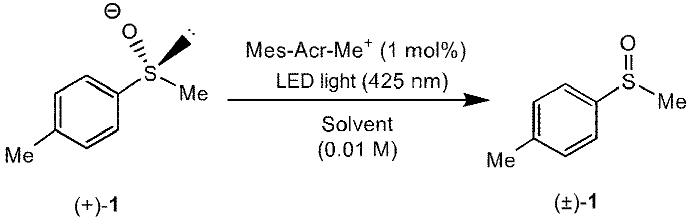

An optically active sulfoxide compound ((+)-1) (3.08 mg, 0.02 mmol) was dissolved in a variety of solvents (2.0 mL). To the obtained solution, 10-methyl-9-(2,4,6-trimethylphenyl)acridinium perchlorate (Mes-Acr-Me⁺) (82.3 mg, 0.2 mmol, 1 mol%) was added, and the obtained mixture was irradiated with light using an 18 W LED light (wavelength 425 nm). During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

**[Table 4]**

| Entry | Solvent | Time required for racemization | Enantiomer ratio after 10 min |
|---|---|---|---|
| 1 | Acetonitrile | 6 min | 50:50 |
| 2 | Ethyl acetate | > 10 min | 72:28 |
| 3 | Dichloromethane | < 10 min | 50:50 |

As shown in Table 4, photoracemization could proceed even when any solvent was used, and the photoracemization reaction was fast particularly when acetonitrile was used.

### <Reference Example 5: preparation of solid photosensitizer in which TPT⁺ is covalently bound to carrier>

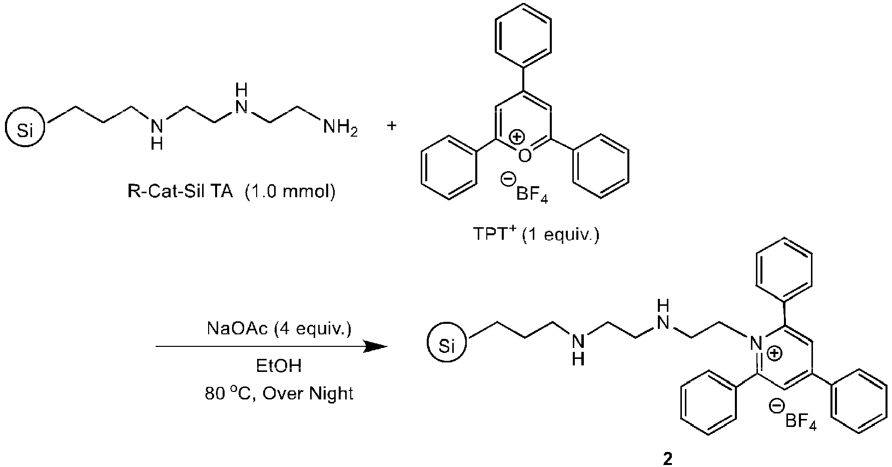

To an ethanol solution (10 mL) containing 2,4,6-triphenylpyrylium tetrafluoroborate (TPT⁺) (0.40 g, 1.0 mmol, 1.0 equiv.), sodium acetate (0.32 g, 4.0 mmol, 4.0 equiv.) and R-Cat-Sil TA (1.0 g, 1.0 mmol, 1.0 equiv.; manufactured by KANTO CHEMICAL CO., INC.) were added, and the obtained mixture was stirred overnight at 80°C. The mixture was cooled to 0°C and then filtered. The obtained residue was washed with methanol and dichloromethane and then dried to obtain a solid photosensitizer 2 (1.12 g).

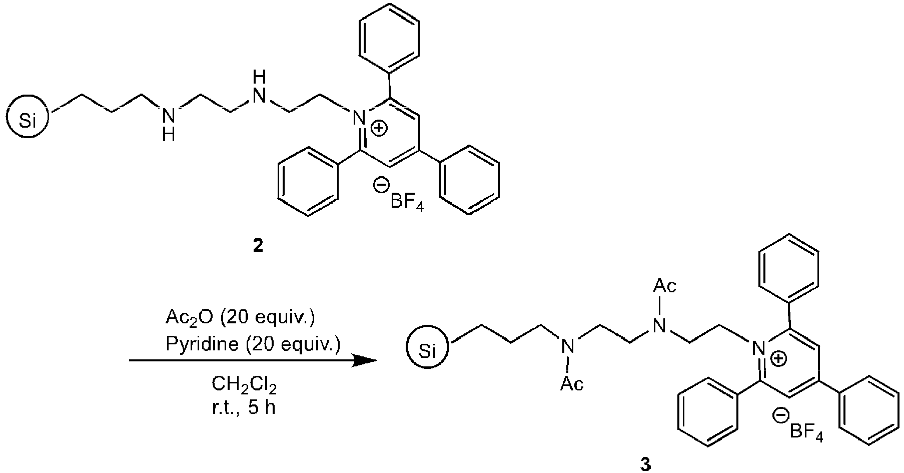

To a dichloromethane solution (10 mL) containing the solid photosensitizer 2 (0.50 g), acetic anhydride (0.90 mL, 10.0 mmol, 20 equiv.) and pyridine (0.8 mL, 10.0 mmol, 20 equiv.) were added, and the obtained mixture was stirred at room temperature for 5 hours. After filtration, the obtained residue was washed with dichloromethane and dried to obtain a solid photosensitizer 3 (478 mg).

### <Reference Example 6: consideration of photoracemization using solid photosensitizer 2 or solid photosensitizer 3>

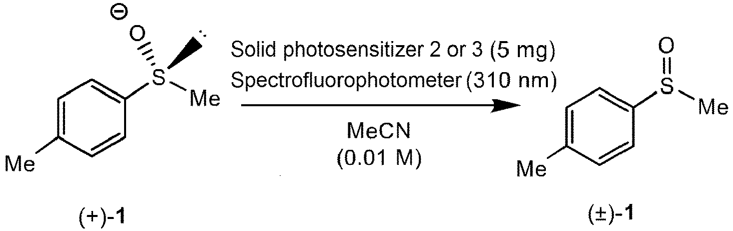

To an acetonitrile solution (1.0 mL, 0.01 M) containing an optically active sulfoxide compound ((+)-1) (1.54 mg, 0.01 mmol), the solid photosensitizer 2 (5 mg) or solid photosensitizer 3 (5 mg) was added, and the obtained mixture was irradiated with light using a spectrofluorophotometer (wavelength 310 nm, RF-5300PC manufactured by SHIMADZU CORPORATION). During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

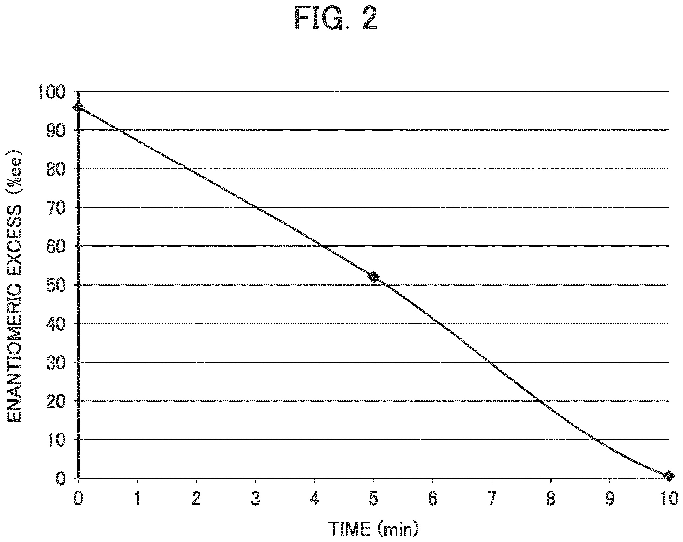

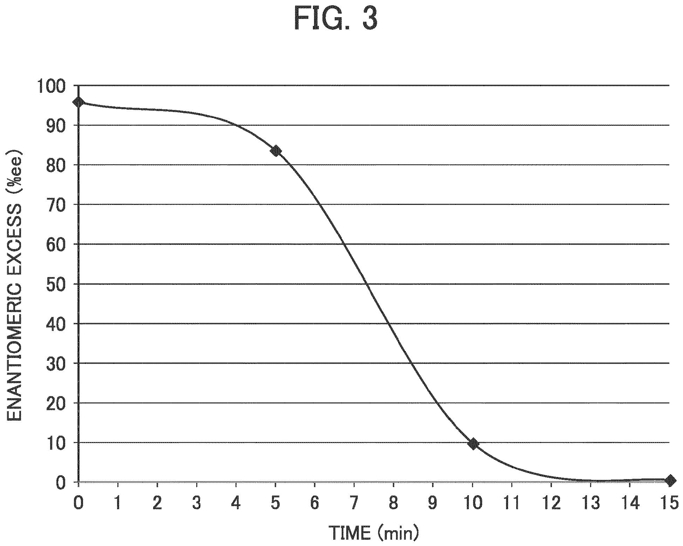

Changes in the enantiomeric excess with time when using the solid photosensitizer 2 and the solid photosensitizer 3 are shown in Fig. 2 and 3 respectively. As shown in Fig. 2 and Fig. 3, the photoracemization reaction could be allowed to efficiently proceed even when a photosensitizer is immobilized to a carrier.

### <Reference Example 7: preparation of solid photosensitizer in which 9,10-DCA is covalently bound to carrier>

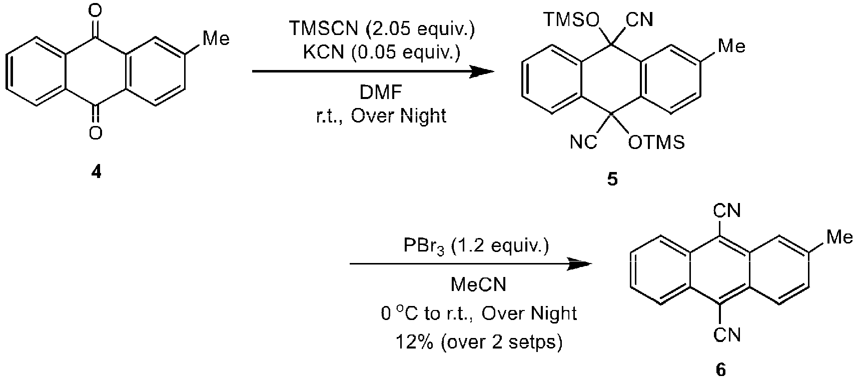

To a N,N-dimethylformamide solution (6.7 mL) containing 2-methylanthraquinone (1.50 g, 6.75 mmol) and potassium cyanide (22.0 mg, 0.38 mmol, 0.05 equiv.), trimethylsilyl cyanide (1.71 mL, 13.9 mmol, 2.05 equiv.) was added under an argon flow, and the obtained mixture was stirred overnight at room temperature. After completion of the reaction, acetonitrile (30 mL) and then phosphorus tribromide (1.54 mL, 16.2 mmol, 1.2 equiv.) were added thereto, and the obtained mixture was stirred overnight at room temperature. Dichloromethane was added thereto to stop the reaction, followed by filtration. The residue obtained by condensing the filtrate was purified by column chromatography (eluent: hexane/dichloromethane = 1/1) to obtain a compound 6 (200 mg, 0.826 mmol, yield: 120).

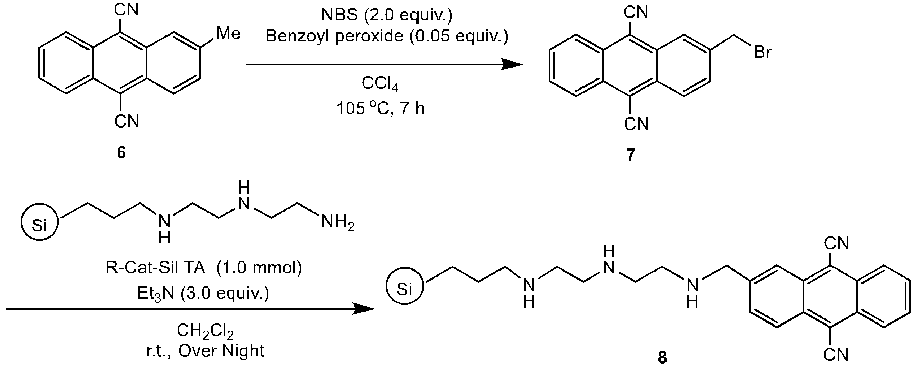

To a carbon tetrachloride solution (10 mL) containing the compound 6 (207 mg, 0.86 mmol), N-bromosuccinimide (290 mg, 1.90 mmol, 2.0 equiv.) and benzoyl peroxide (10.0 mg, 0.04 mmol, 0.05 equiv.) were added under an argon flow, and the obtained mixture was stirred under heat reflux for 7 hours. After completion of the reaction, the residue obtained by condensation was purified by column chromatography (eluent: hexane/ethyl acetate = 20/1) to obtain a compound 7. The obtained compound 7 was dissolved in dichloromethane (9.3 mL), and R-Cat-Sil TA (1.0 g, 1.0 mmol, 1.1 equiv.; manufactured by KANTO CHEMICAL CO., INC.) and triethylamine (0.42 mL, 3.00 mmol, 3 equiv.) were added thereto under an argon flow, and the obtained mixture was stirred overnight at room temperature. After filtration, the residue was washed with water, methanol, dichloromethane and ethyl acetate, and dried to obtain a solid photosensitizer 8 (1.23 g).

### <Reference Example 8: preparation of solid photosensitizer in which TPT⁺ is ionically bound to carrier>

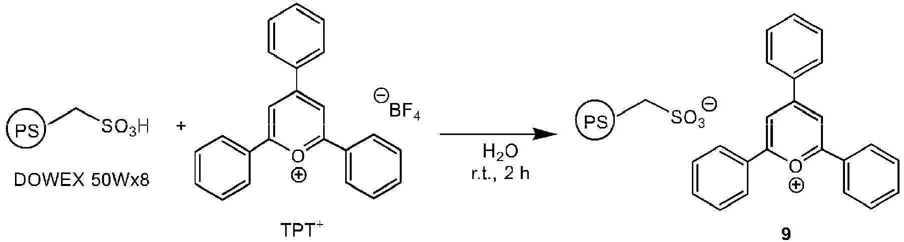

To an aqueous solution (60 mL) containing 2,4,6-triphenylpyrylium tetrafluoroborate (TPT⁺) (0.20 g, 0.50 mol), DOWEX 50W x8 (2.0 g; manufactured by FUJIFILM Wako Pure Chemical Corporation) was added, and the obtained mixture was stirred at room temperature for 17 hours. After filtration, the residue was washed with water, methanol, dichloromethane and acetonitrile, and dried to obtain a solid photosensitizer 9 (1.70 g).

### <Reference Example 9: consideration of photoracemization using solid photosensitizer 9>

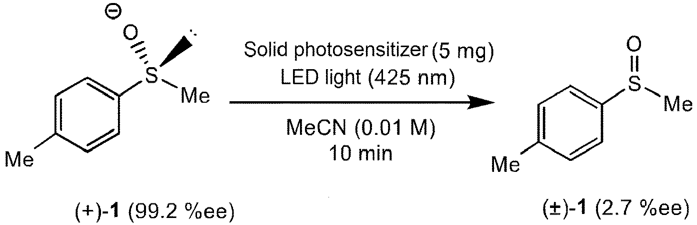

To an acetonitrile solution (1.0 mL, 0.01 M) containing an optically active sulfoxide compound ((+)-1) (1.54 mg, 0.01 mmol), the solid photosensitizer 9 (5 mg) was added, and the obtained mixture was irradiated with light using an 18 W LED light (wavelength 425 nm). During light irradiation, sampling (20 mL) was carried out over time to calculate the enantiomeric excess thereof by chiral HPLC. The chiral HPLC conditions are as described below.

### -Chiral HPLC conditions-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

The enantiomeric excess was reduced from 99.2% ee to 2.7% ee by irradiation with light at a wavelength of 425 nm in the presence of the solid photosensitizer 9 for 10 minutes, and it was observed that the photoracemization reaction efficiently proceeded.

### <Reference Example 10: consideration of substrate generality>

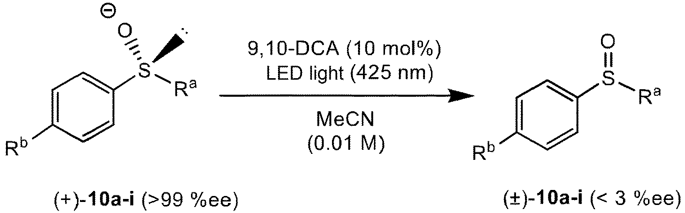

To an acetonitrile solution (3.0 mL, 0.01 M) containing an optically active sulfoxide compound ((+)-10a) (4.30 mg, 0.03 mmol), 9,10-dicyanoanthracene (9,10-DCA) (0.70 mg, 0.003 mmol, 10 mol%) was added, and the obtained mixture was irradiated with light using an 18 W LED light (wavelength 425 nm). During light irradiation, sampling (20 mL) was carried out over time to calculate the enantiomeric excess thereof by chiral HPLC. The same operation as above was carried out except that (+)-10b to 10i were used in place of (+)-10a. The chiral HPLC conditions are as described below.

### -Chiral HPLC conditions (10a to 10d, 10f to 10h)-

Column: CHIRALPACK IH (10 mm x 250 mm, particle diameter 5 mm) (manufactured by Daicel Corporation),
Mobile phase: hexane/isopropanol = 60/40,
Flow rate: 0.5 mL/min, and
Detection: 254 nm.

### -Chiral HPLC conditions (10e)-

Column: CHIRALPACK IA (10 mm x 250 mm, particle diameter 5 mm) (manufactured by Daicel Corporation),
Mobile phase: hexane/ethanol = 50/50,
Flow rate: 0.5 mL/min, and
Detection: 254 nm.

### -Chiral HPLC conditions (10i)-

Column: CHIRALPACK IH (10 mm x 250 mm, particle diameter 5 mm) (manufactured by Daicel Corporation),
Mobile phase: hexane/isopropanol = 80/20,
Flow rate: 0.5 mL/min, and
Detection: 254 nm.

**[Table 5]**

| Entry | | R^{a} | R^{b} | Time required for racemization |
|---|---|---|---|---|
| 1 | **10a** | Me | H | 1 min |
| 2 | **10b** | Et | H | 0.5 min |
| 3 | **10c** | 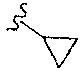 | H | 0.5 min |
| 4 | **10d** | -CHCH₂ | H | 5 min |
| 5 | **10e** | 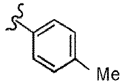 | H | 1 min |
| 6 | **10f** | -CH₂COOEt | H | 45 min |
| 7 | **10g** | Me | F | 2 min |
| 8 | **10h** | Me | Cl | 2 min |
| 9 | **10i** | Me | -OMe | 0.5 min |

As shown in Table 5, photoracemization could proceed even when any sulfoxide compound was used.

### EXPLANATION OF REFERENCE NUMERALS

1: Enantiomer preparation system, 10: optical resolution part, and 20: light irradiation part.

## Claims

1. A method for selectively preparing one of enantiomers of a sulfoxide compound having a sulfur atom in a sulfoxide group as an asymmetric center in an enantiomer preparation system including an optical resolution part (10) to optically resolve an enantiomer mixture of the sulfoxide compound into one enantiomer and the other enantiomer, and a light irradiation part (20) to irradiate the other enantiomer obtained in the optical resolution part with light to racemize the enantiomer, the method comprising:
Step A for optically resolving an enantiomer mixture of the sulfoxide compound in the optical resolution part (10) into one enantiomer and the other enantiomer, isolating the one enantiomer and sending the other enantiomer to the light irradiation part (20),
Step B for irradiating the other enantiomer obtained in Step A or Step C in the light irradiation part (20) with light to racemize the enantiomer and sending the enantiomer mixture of the sulfoxide compound by racemization to the optical resolution part (10) again with a recirculating system, and
Step C for optically resolving the enantiomer mixture of the sulfoxide compound obtained in Step B into the one enantiomer and the other enantiomer in the optical resolution part (10), wherein the enantiomer mixture of the sulfoxide compound is optically resolved by a chromatography method using a chiral column in both Step A and Step C, and wherein Step B and Step C are repeated, wherein in Step B the other enantiomer is irradiated with light in the presence of a photosensitizer to racemize the enantiomer, wherein the photosensitizer is a solid photosensitizer immobilized to a carrier.

2. The method for preparing an enantiomer according to claim 1, wherein the chromatography method using a chiral column is a recycle HPLC using a chiral column.

3. The method for preparing an enantiomer according to claim 2, wherein in step B the other enantiomer coming from a column outlet of the chiral column of the recycle HPLC in step A is irradiated with light to photoracemize the other enantiomer, and the resulting enantiomer mixture is returned to a column inlet of the chiral column of the recycle HPLC.

4. The method for preparing an enantiomer according to any one of claims 1 to 3, wherein the sulfoxide compound is a compound represented by formula (1) below: 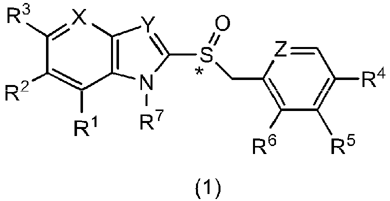
wherein, R¹, R² and R³ are each independently a hydrogen atom, a halogen atom, a cyano group, a nitro group, a hydroxy group, a carboxy group, an alkyl group which optionally has a substituent, an alkoxy group which optionally has a substituent, an alkoxycarbonyl group, an aryl group, an aryloxy group, an acyl group, an acyloxy group or a heteroaryl group,
R⁴, R⁵ and R⁶ are each independently a hydrogen atom, an alkyl group which optionally has a substituent, an alkoxy group which optionally has a substituent, or an amino group which optionally has a substituent,
R⁷ is a hydrogen atom, a hydrocarbon group which optionally has a substituent, an acyl group or an acyloxy group,
X, Y and Z are each independently a nitrogen atom or CH, and
* represents an asymmetric center.

5. An enantiomer preparation system for use in the method for preparing an enantiomer according to any one of claims 1 to 4, the enantiomer preparation system comprising:
an optical resolution part (10) provided with a chiral column configured to optically resolve the enantiomer mixture of the sulfoxide compound into one enantiomer and the other enantiomer, and
a light irradiation part (20) configured to irradiate the other enantiomer obtained in the optical resolution part with light to racemize the enantiomer, wherein the optical resolution part (10) optically resolves the enantiomer mixture of the sulfoxide compound obtained in the light irradiation part into the one enantiomer and the other enantiomer, further including a recirculating system which is configured to send an enantiomer mixture of the sulfoxide compound obtained in the light irradiation part (20) to the optical resolution part (10) again, wherein in the light irradiation part (20) a solid photosensitizer immobilized to a carrier is present.

6. The enantiomer preparation system according to claim 5, wherein the optical resolution part (10) provided with a chiral column is a recycle HPLC apparatus including a chiral column.

## Patentansprüche

1. Verfahren zur selektiven Herstellung eines Enantiomers einer Sulfoxidverbindung mit einem Schwefelatom in einer Sulfoxidgruppe als asymmetrisches Zentrum in einem System zur Herstellung eines Enantiomers, das einen optischen Auflöseteil (10) zum optischen Auflösen einer Enantiomermischung der Sulfoxidverbindung in ein Enantiomer und das andere Enantiomer und einen Lichtbestrahlungsteil (20) zum Bestrahlen des anderen Enantiomers, das im optischen Auflöseteil erhalten wird, zum Racemisieren des Enantiomers aufweist, wobei das Verfahren umfasst:
Schritt A zum optischen Auflösen einer Enantiomermischung der Sulfoxidverbindung im optischen Auflöseteil (10) in ein Enantiomer und das andere Enantiomer, Isolieren des einen Enantiomers und Überführen des anderen Enantiomers an den Lichtbestrahlungsteil (20),
Schritt B zum Bestrahlen des anderen Enantiomers, das in Schritt A oder Schritt C im Lichtbestrahlungsteil (20) erhalten wird, mit Licht zum Racemisieren des Enantiomers und erneutes Überführen der Enantiomermischung der Sulfoxidverbindung durch Racemisierung an den optischen Auflöseteil (10) mit einem Kreislaufsystem und
Schritt C zum optischen Auflösen der Enantiomermischung der Sulfoxidverbindung, die in Schritt B erhalten wird, in das eine Enantiomer und das andere Enantiomer im optischen Auflöseteil (10), wobei die Enantiomermischung der Sulfoxidverbindung mit einem Chromatografieverfahren unter Verwendung einer chiralen Säule sowohl in Schritt A als auch Schritt C optisch aufgelöst wird, und wobei Schritt B und Schritt C wiederholt werden, wobei das andere Enantiomer in Schritt B in Gegenwart eines Photosensibilisators mit Licht bestrahlt und so das Enantiomer racemisiert wird, wobei der Photosensibilisator ein fester Photosensibilisator ist, der an einem Träger immobilisiert ist.

2. Verfahren zur Herstellung eines Enantiomers nach Anspruch 1, wobei das Chromatographieverfahren unter Verwendung einer chiralen Säule eine Recycling-HPLC unter Verwendung einer chiralen Säule ist.

3. Verfahren zur Herstellung eines Enantiomers nach Anspruch 2, wobei das andere Enantiomer, das aus einem Säulenauslass der chiralen Säule der Recycling-HPLC in Schritt A stammt, in Schritt B mit Licht bestrahlt und so das andere Enantiomer unter Lichteinwirkung racemisiert wird und die entstehende Enantiomermischung zu einem Säuleneinlass der chiralen Säule der Recycling-HPLC zurückgeleitet wird.

4. Verfahren zur Herstellung eines Enantiomers nach einem der Ansprüche 1 bis 3, wobei die Sulfoxidverbindung eine Verbindung ist, die mit der nachstehenden Formel (1) wiedergegeben ist: 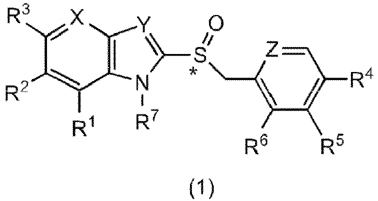
in der R¹, R² und R³ jeweils unabhängig ein Wasserstoffatom, ein Halogenatom, eine Cyanogruppe, eine Nitrogruppe, eine Hydroxygruppe, eine Carboxygruppe, eine Alkylgruppe, die optional einen Substituenten aufweist, eine Alkoxygruppe, die optional einen Substituenten aufweist, eine Alkoxycarbonylgruppe, eine Arylgruppe, eine Aryloxygruppe, eine Acylgruppe, eine Acyloxygruppe oder eine Heteroarylgruppe ist,
R⁴, R⁵ und R⁶ jeweils unabhängig ein Wasserstoffatom, eine Alkylgruppe, die optional einen Substituenten aufweist, eine Alkoxygruppe, die optional einen Substituenten aufweist, oder eine Aminogruppe ist, die optional einen Substituenten aufweist,
R⁷ ein Wasserstoffatom, eine Kohlenwasserstoffgruppe, die optional einen Substituenten aufweist, eine Acylgruppe oder eine Acyloxygruppe ist,
X, Y und Z jeweils unabhängig ein Stickstoffatom oder CH ist, und
* ein asymmetrisches Zentrum darstellt.

5. System zur Herstellung eines Enantiomers zur Verwendung in dem Verfahren zur Herstellung eines Enantiomers nach einem der Ansprüche 1 bis 4, wobei das System zur Herstellung eines Enantiomers Folgendes umfasst:
einen optischen Auflöseteil (10), der mit einer chiralen Säule versehen ist, die so eingerichtet ist, dass sie die Enantiomermischung der Sulfoxidverbindung optisch in ein Enantiomer und das andere Enantiomer auflöst, und
einen Lichtbestrahlungsteil (20), der so eingerichtet ist, dass er das andere Enantiomer, das im optischen Auflöseteil erhalten wird, mit Licht bestrahlt und so das Enantiomer racemisiert wird, wobei der optische Auflöseteil (10) die Enantiomermischung der Sulfoxidverbindung, die im Lichtbestrahlungsteil erhalten wird, optisch in das eine Enantiomer und das andere Enantiomer auflöst, ferner aufweisend ein Kreislaufsystem, das so eingerichtet ist, dass es eine Enantiomermischung der Sulfoxidverbindung, die im Lichtbestrahlungsteil (20) erhalten wird, erneut an den optischen Auflöseteil (10) überführt, wobei im Lichtbestrahlungsteil (20) ein fester Photosensibilisator vorliegt, der an einem Träger immobilisiert ist.

6. System zur Herstellung eines Enantiomers nach Anspruch 5, wobei der optische Auflöseteil (10), der mit einer chiralen Säule versehen ist, ein Recycling-HPLC-Gerät ist, das eine chirale Säule aufweist.

## Revendications

1. Procédé de préparation sélective d'un des énantiomères d'un composé sulfoxyde ayant un atome de soufre dans un groupe sulfoxyde comme centre asymétrique dans un système de préparation d'énantiomères comprenant une partie de résolution optique (10) pour résoudre optiquement un mélange d'énantiomères du composé sulfoxyde en un énantiomère et l'autre énantiomère et une partie d'irradiation de lumière (20) pour irradier l'autre énantiomère obtenu dans la partie de résolution optique avec de la lumière pour racémiser l'énantiomère, le procédé comprenant :
une étape A pour résoudre optiquement un mélange d'énantiomères du composé sulfoxyde dans la partie de résolution optique (10) en un énantiomère et l'autre énantiomère, isolant l'un énantiomère et envoyant l'autre énantiomère à la partie d'irradiation de lumière (20),
une étape B pour irradier l'autre énantiomère obtenu dans l'étape A ou l'étape C dans la partie d'irradiation de lumière (20) avec de la lumière pour racémiser l'énantiomère et envoyer le mélange d'énantiomères du composé sulfoxyde par racémisation à la partie de résolution optique (10) à nouveau avec un système de recirculation et
une étape C pour résoudre optiquement le mélange d'énantiomères du composé sulfoxyde obtenu dans l'étape B en l'un énantiomère et l'autre énantiomère dans la partie de résolution optique (10), le mélange d'énantiomères du composé sulfoxyde étant résolu optiquement par un procédé de chromatographie en utilisant une colonne chirale dans les deux étape A et étape C et l'étape B et l'étape C étant répétées, dans l'étape B l'autre énantiomère étant irradié avec de la lumière en présence d'un photosensibilisateur pour racémiser l'énantiomère, le photosensibilisateur étant un photosensibilisateur solide immobilisé sur un support.

2. Procédé de préparation d'un énantiomère selon la revendication 1, dans lequel le procédé de chromatographie utilisant une colonne chirale est une CLHP de recyclage utilisant une colonne chirale.

3. Procédé de préparation d'un énantiomère selon la revendication 2, dans lequel dans l'étape B l'autre énantiomère provenant d'une sortie de colonne de la colonne chirale de la CLHP de recyclage dans l'étape A est irradié avec de la lumière pour photoracémiser l'autre énantiomère et le mélange d'énantiomères résultant est retourné à une entrée de colonne de la colonne chirale de la CLHP de recyclage.

4. Procédé de préparation d'un énantiomère selon l'une quelconque des revendications 1 à 3, dans lequel le composé sulfoxyde est représenté par la formule (1) ci-dessous : 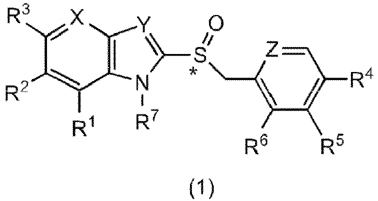
dans laquelle, R¹, R² et R³ sont chacun indépendamment un atome d'hydrogène, un atome d'halogène, un groupe cyano, un groupe nitro, un groupe hydroxy, un groupe carboxy, un groupe alkyle qui éventuellement a un substituant, un groupe alcoxy qui éventuellement a un substituant, un groupe alcoxycarbonyle, un groupe aryle, un groupe aryloxy, un groupe acyle, un groupe acyloxy ou un groupe hétéroaryle, R⁴, R⁵ et R⁶ sont chacun indépendamment un atome d'hydrogène, un groupe alkyle qui éventuellement a un substituant, un groupe alcoxy qui éventuellement a un substituant ou un groupe amino qui éventuellement a un substituant,
R⁷ est un atome d'hydrogène, un groupe hydrocarbure qui éventuellement a un substituant, un groupe acyle ou un groupe alcoxy,
X, Y et Z sont chacun indépendamment un atome d'azote ou un groupe CH et
* représente un centre asymétrique.

5. Système de préparation d'énantiomère destiné à être utilisé dans le procédé pour préparer un énantiomère selon l'une quelconque des revendications 1 à 4, le système de préparation d'énantiomère comprenant :
une partie de résolution optique (10) fournie avec une colonne chirale configurée pour résoudre optiquement le mélange d'énantiomères en un énantiomère et l'autre énantiomère et
une partie d'irradiation de lumière (20) configurée pour irradier l'autre énantiomère obtenu dans la partie de résolution optique avec de la lumière pour racémiser l'énantiomère, la partie de résolution optique (10) résolvant optiquement le mélange d'énantiomères du composé sulfoxyde obtenu dans la partie d'irradiation de lumière en l'un énantiomère et l'autre énantiomère, comprenant en outre un système de recirculation qui est configuré pour envoyer un mélange d'énantiomères du composé sulfoxyde obtenu dans la partie d'irradiation de lumière (20) à la partie de résolution optique (10) à nouveau, dans la partie d'irradiation de lumière (20) un photosensibilisateur solide immobilisé sur un support étant présent.

6. Système de préparation d'énantiomères selon la revendication 5, dans lequel la partie de résolution optique (10) fournie avec une colonne chirale est un appareil de CLHP de recyclage comprenant une colonne chirale.
